# EUROPEAN PATENT APPLICATION

(11) **EP 3 960 627 A1**
(43) Date of publication of application: **02.03.2022**
(21) Application number: 21175793.5
(22) Date of filing: 25.05.2021
(51) Int. Cl.: B64D 11/04, A61L 2/04, A61L 2/10, A61L 2/20

(54) **LOOSE OBJECTS AND PROTECTIVE GEAR DISINFECTION GALLEY INSERT**

(30) Priority: 22.05.2020 US 202063028941 P; 27.05.2020 US 202063030461 P
(71) Applicant: Koninklijke Fabriek Inventum B.V., 3439 MG Nieuwegein (NL)
(72) Inventor: GONZALEZ, Arnau Castillo, 3607AK Maarssen (NL)
(74) Representative: Dehns

(57) **Abstract**

A galley insert assembly (108) for an aircraft galley comprises an insert body (110) including a plurality of insert walls (112) defining an interior space (114) therein. The insert walls are configured to interface with a galley bay (104) of an aircraft with the insert body inserted into the galley bay. A disinfection system (124) is included within the interior space and a door is operatively connected to the insert walls.

## Description

### CROSS-REFRENCE TO RELATED APPLICATIONS

This application claims priority to and the benefit of U.S. Provisional Patent Application No. 63/028,941 filed May 22, 2021, and U.S. Provisional Patent Application No. 63/030,461 filed May 27, 2021.

### BACKGROUND

### 1. Field

The present disclosure relates generally to disinfection and sanitization of loose objects and protective gear such as face masks, and more particularly to such disinfection and sanitization aboard aircraft.

### 2. Description of Related Art

The COVID-19 outbreak has raised the demands of disinfection in aircraft cabins and the objects within. For example, it has provoked a shortage of protective masks due to the sudden high demand. There is an ongoing need for improved systems and methods to decontaminate loose objects, including masks and other protective equipment, aboard aircraft providing service to the crew, ground personnel, and passengers. This disclosure provides a solution for this need.

### SUMMARY

A galley insert assembly for an aircraft galley comprises an insert body including a plurality of insert walls defining an interior space therein. The insert walls are configured to interface with a galley bay of an aircraft with the insert body inserted into the galley bay. A disinfection system is included within the interior space and a door is operatively connected to the insert walls. The door is configured to open and close relative to the insert walls to selectively enclose the interior space, and allow access to the interior space. The interior space can be large enough to accommodate a plurality of personal protective equipment (PPE) masks.

The disinfection system can be electrically connected to an electrical connector extending outside of at least one of the insert walls and configured to electrically connect the insert body to a reciprocal connector in the galley bay for providing aircraft power to the disinfection system.

The disinfection system can include a heating element which can be configured to heat objects within the interior space to a required disinfection temperature. The disinfection system can include a distributor configured to distribute at least one of a hydrogen peroxide bath and/or an Ozone bath over objects within the interior space.

The disinfection system can include a UV illumination system configured to illuminate surfaces of objects in the interior space with UV illumination. In embodiments, the UV illumination system can include a plurality of UVC illuminators configured to emit UVC illumination.

An aircraft galley comprises a galley interior defining a plurality of galley bays each configured to receive a galley insert and a galley insert assembly. The galley insert assembly includes an insert body including a plurality of insert walls defining an interior space therein. The insert walls interface with a first one of the galley bays of an aircraft with the insert body inserted into the first galley bay. A disinfection system is within the interior space and a door is operatively connected to the insert walls. The door is configured to open and close relative to the insert walls to selectively enclose the interior space, and allow access to the interior space.

A method comprises placing objects inside a galley insert in a galley of an aircraft and activating a disinfection system within the galley insert to disinfect the objects. Activating the disinfection system can include any or all of: activating a UV illumination system for a threshold period of time, activating a UV illumination system for a threshold period of time and simultaneously or subsequently activating a heating element of a second threshold period of time, and/or activating at least one of a hydrogen peroxide bath and/or an Ozone bath for a threshold period of time. The method can further include retrofitting a galley insert with the disinfection system.

These and other features of the systems and methods of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:
Fig. 1 is a schematic perspective view of an embodiment of an aircraft galley constructed in accordance with the present disclosure, showing a plurality of galley inserts; and
Fig. 2 is a schematic perspective view of an embodiment of an aircraft galley insert having a disinfection system constructed in accordance with at least one aspect of the present disclosure.

### DETAILED DESCRIPTION

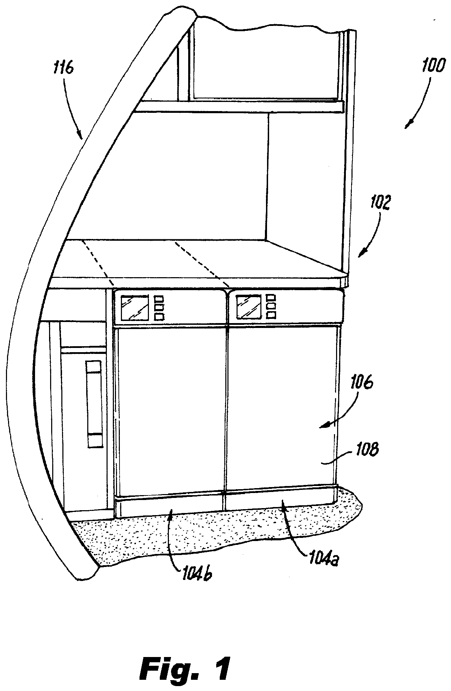

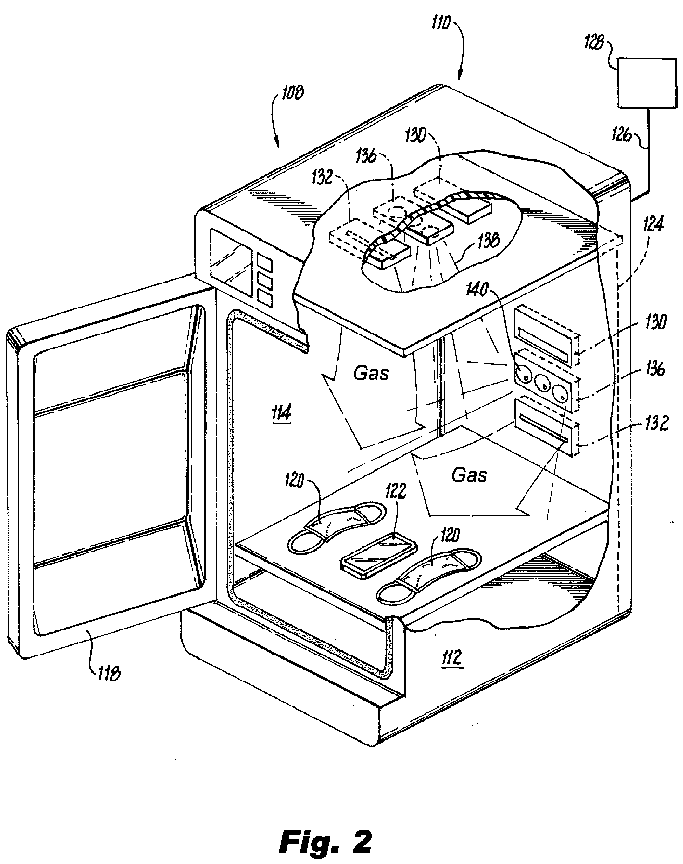

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. Other embodiments of systems in accordance with the disclosure, or aspects thereof, are provided in Fig. 2, as will be described. The systems and methods described herein can be used to disinfect and decontaminate loose items on demand, e.g. within aircraft galleys.

Referring to Fig. 1, an aircraft galley 100 comprises a galley interior 102 defining a plurality of galley bays 104a, 104b each configured to receive a galley insert 106 and a galley insert assembly 108. The galley insert assembly 108 includes an insert body 110 including a plurality of insert walls 112 defining an interior space 114 therein. The insert walls 112 interface with a first one of the galley bays 104a of an aircraft 116 with the insert body 110 inserted into the first galley bay 104a. A door 118 is operatively connected to the insert walls 112, configured to open and close relative to the insert walls 112 to selectively enclose the interior space 114, and allow access to the interior space 114. The interior space 114 can be large enough to accommodate a plurality of personal protective equipment (PPE) masks 120, or other personal items that are high touch point surfaces, e.g. personal electronic device 122.

Referring now to Fig. 2, the disinfection system 124 is disposed within the interior space 114 to disinfect the PPE 120 and devices 122. The disinfection system 124 can be electrically connected to an electrical connector 126 extending outside of at least one of the insert walls 112 and configured to electrically connect the insert body 110 to a reciprocal connector 128 in the galley bay 104 for providing aircraft power to the disinfection system 124.

In certain embodiments, the disinfection system 124 can include a heating element 130 for heating the objects 120, 122 within the interior space 112 to a required disinfection temperature (e.g. 150°C or greater). For example, the disinfection system 124 can heat the objects 120, 122 for any suitable threshold period of time until disinfection is complete (e.g. about one hour). In certain embodiments, the disinfection system 124 can include a distributor 132 configured to distribute at least one of a hydrogen peroxide bath and/or an Ozone bath 134 over objects within the interior space 112. For example, a concentrated hydrogen peroxide vapor bath 134 can be distributed over the objects 120, 122 for any suitable threshold period of time (e.g. two and a half hours).

In certain embodiments the disinfection system 124 can include a UV illumination system 136 configured to illuminate surfaces of the objects 120, 122 in the interior space 112 with UV illumination 138. For example the UV illumination system 136 can include a plurality of UVC illuminators 140 configured to emit UVC illumination. The UV illumination system can be activated for any suitable threshold period of time needed to disinfect the objects 120, 122 (e.g. about eight minutes). It is contemplated that the disinfection system can include any one or all of the heating element 130, distributor 132, and/or UV illumination system 136 in any suitable combination as needed for the specific object in the interior space 112.

A method comprises placing objects 120, 122 inside the galley insert 106 in an aircraft galley 100 and activating the disinfection system 124 within the galley insert 106 for any suitable threshold period of time to disinfect the objects 120, 122 . Activating the disinfection system 124 can include any or all of: activating the UV illumination system 136 for a threshold period of time, activating the UV illumination system 136 for a first threshold period of time and simultaneously or subsequently activating the heating element 130 for a second threshold period of time, and/or activating at least one of a hydrogen peroxide bath and/or an Ozone bath 134 for a threshold period of time. In certain embodiments, the method can further include retrofitting galley insert 106 with the disinfection system 124.

The methods and systems of the present disclosure, as described above and shown in the drawings, provide for improved means for disinfecting and decontaminating loose objects in an aircraft cabin, for crew and passengers as an additional service. Improved on demand disinfection can minimize the spread of contaminants within an aircraft cabin, for example during a flight. While the apparatus and methods of the subject disclosure have been shown and described, those skilled in the art will readily appreciate that changes and/or modifications may be made thereto without departing from the scope of the invention as defined by the claims.

## Claims

1. A galley insert assembly for an aircraft galley comprising:
an insert body (110) including a plurality of insert walls (112) defining an interior space (114) therein, wherein the insert walls are configured to interface with a galley bay (104a, 104b) of an aircraft with the insert body inserted into the galley bay;
a disinfection system (124) within the interior space; and
a door (118) operatively connected to the insert walls, wherein the door is configured to open and close relative to the insert walls to selectively enclose the interior space, and allow access to the interior space.

2. The assembly as recited in claim 1, wherein the disinfection system (124) is electrically connected to an electrical connector (126) extending outside of at least one of the insert walls and configured to electrically connect the insert body to a reciprocal connector (128) in the galley bay, for providing aircraft power to the disinfection system.

3. The assembly as recited in claim 1 or 2, wherein the disinfection system includes a heating element (130) configured to heat objects within the interior space to a required temperature of disinfection.

4. The assembly as recited in any preceding claim, wherein the disinfection system includes a distributor (132) configured to distribute at least one of a hydrogen peroxide bath and/or an Ozone bath (134) over objects (120, 122) within the interior space.

5. The assembly as recited in any preceding claim, wherein the disinfection system includes a UV illumination system (136) configured to illuminate surfaces of objects in the interior space with UV illumination.

6. The assembly as recited in claim 5, wherein the UV illumination system includes a plurality of UVC illuminators (140) configured to emit UVC illumination.

7. The assembly as recited in any preceding claim, wherein the interior space is large enough to accommodate a plurality of personal protective equipment, PPE, masks (120).

8. An aircraft galley comprising:
a galley interior (102) defining a plurality of galley bays (104a, 104b) each configured to receive a galley insert (106); and
a galley insert assembly (108) as claimed in any preceding claim:
wherein the insert walls (112) interface with a first one of the galley bays (104a) with the insert body inserted into the first galley bay.

9. A method comprising:
placing objects (120, 122) inside a galley insert (106) in a galley of an aircraft; and
activating a disinfection system (124) within the galley insert to disinfect the objects.

10. The method as recited in claim 9, wherein activating the disinfection system includes activating a UV illumination system for a threshold period of time.

11. The method as recited in claim 9 or 10, wherein activating the disinfection system includes activating a UV illumination system for a first threshold period of time and simultaneously or subsequently activating a heating element (130) for a second threshold period of time.

12. The method as recited in claim 9, 10 or 11, wherein activating the disinfection system includes activating at least one of a hydrogen peroxide bath and/or an Ozone bath (134) for a threshold period of time.

13. A method comprising, retrofitting a galley insert with a disinfection system.
